# EUROPEAN PATENT APPLICATION

(11) **EP 4 190 398 A1**
(43) Date of publication of application: **07.06.2023**
(21) Application number: 21211891.3
(22) Date of filing: 02.12.2021
(51) Int. Cl.: A61N 1/375, A61N 1/05

(54) **ANCHORING DEVICE FOR ANCHORING AN IMPLANTABLE MEDICAL DEVICE ON TISSUE**

(71) Applicant: BIOTRONIK SE & Co. KG, 12359 Berlin (DE)
(72) Inventor: Klaus, Sebastian, 8424 Embrach (CH); Henschel, Martin, 13125 Berlin (DE); Hughes, Devan, Tualatin, 97062 (US); Muessig, Dirk, West Linn, 97068 (US); Lang, Volker, 12161 Berlin (DE)
(74) Representative: Biotronik Corporate Services SE

(57) **Abstract**

An anchoring device (2) for anchoring an implantable medical device (1) on tissue comprises a first fixation member (20) having a first fixation arrangement (201) for engaging with tissue, a second fixation member (22) having a second fixation arrangement (221) for engaging with tissue, the second fixation member (22) being laterally offset with respect to the first fixation member (20) along a lateral direction (L), and an elastically deformable connection structure (21) operatively connecting said first fixation member (20) and said second fixation member (22) with each other. The connection structure (21) is configured to tension said first fixation member (20) and said second fixation member (22) with respect to one another along the lateral direction (L) for anchoring the anchoring device (2) on tissue by said first fixation arrangement (201) and said second fixation arrangement (221).

## Description

The instant invention generally relates to an anchoring device for anchoring an implantable medical device on tissue and a method for implanting an implantable medical device.

An implantable medical device of this kind may for example be an active implantable medical device (in short AIMD) which comprises electronic circuitry and an energy supply for performing a medical function within a patient's heart, such as a leadless pacemaker for implantation into the heart, for example in the right atrium of the heart.

In recent years, leadless pacemakers have received increasing attention. Leadless pacemakers, in contrast to pacemakers implanted subcutaneously using leads extending transvenously into the heart, avoid leads in that the pacemaker device itself is implanted into the heart, the pacemaker having the shape of a generally cylindrical capsule for implantation within a cardiac chamber. Such leadless pacemakers exhibit the inherent advantage of not using leads, which can reduce risks for the patient involved with leads transvenously accessing the heart, such as the risk of pneumothorax, lead dislodgement, cardiac perforation, venous thrombosis, and the like.

When placing an implantable medical device such as a leadless pacemaker device in the human heart, one challenge lies in designing an anchoring device allowing a fixation of the implantable medical device on cardiac tissue such that the implantable medical device is securely received and held to the cardiac tissue. For this, the anchoring device on the one hand shall provide for a secure fastening of the implantable medical device on the cardiac tissue, on the other hand however shall avoid a damaging of cardiac tissue and structures within the cardiac tissue.

A typical anchoring device comprises anchoring members in the shape of tines which, when placing the implantable medical device on cardiac tissue, pierce the cardiac tissue and in this way provide for an active anchoring of the implantable medical device on the cardiac tissue. For placing the implantable medical device on cardiac tissue, the implantable medical device for example is received within a sheathing device of a delivery catheter, the delivery catheter being used to access the human heart and to deliver the implantable medical device towards a location of interest by removing the sheathing device and by in this way placing the implantable medical device on cardiac tissue at the location of interest. Upon delivering the implantable medical device from the sheathing device the anchoring members engage with cardiac tissue to provide for a fastening of the implantable medical device on the cardiac tissue.

US 9,844,659 describes an assembly of an implantable medical device comprising an anchoring device having a set of active fixation tines. The active fixation tines in the set are deployable from a spring-loaded position in which distal ends of the fixation tines point away from the implantable medical device to a hooked position in which the active fixation tines bend back towards the implantable medical device.

US 9,526,891 discloses an implantable pacemaker system including a housing having a proximal end and a distal end, an arrangement of anchoring members being placed on the distal end of the housing for providing for a fastening of the implantable pacemaker system on cardiac tissue.

US 8,700,181 discloses a leadless intracardiac medical device configured to be implanted entirely within a heart of a patient, the device including a housing configured to be securely attached to an interior wall portion of a chamber of the heart, and a stabilizing intra-cardiac device extension connected to the housing. The stabilizing intra-cardiac device extension may include a stabilizer arm or an appendage arm, or an elongated body or a loop member configured to be passively secured within the heart.

There is a general desire for an anchoring device which allows for an easy and reliable anchoring of an implantable medical device on tissue, in particular within the ventricle or the atrium of the heart. There herein is the wish that the anchoring shall be flexibly possible within different locations, wherein it is desirable that an easy repositioning of the implantable medical device is possible while reducing a damaging impact on tissue.

It is an objective of the instant disclosure to provide an anchoring device for anchoring an implantable medical device on tissue and a method for implanting an implantable medical device which allow for an easy and reliable anchoring of the implantable medical device on tissue in particular within a patient's heart, while facilitating a repositioning of the implantable medical device.

This objective is in generally attained by an anchoring device comprises a first fixation member, a second fixation member, the second fixation member being laterally offset with respect to the first fixation member along a lateral direction; and an connection structure operatively connecting said first fixation member and said second fixation member with each other.

The objective is particularly addressed by an anchoring device comprising the features of claim 1.

In one aspect, an anchoring device for anchoring an implantable medical device on tissue comprises a first fixation member having a first fixation arrangement for engaging with tissue; a second fixation member having a second fixation arrangement for engaging with tissue, the second fixation member being laterally offset with respect to the first fixation member along a lateral direction; and an connection structure operatively connecting said first fixation member and said second fixation member with each other, wherein the connection structure is configured to tension said first fixation member and said second fixation member with respect to one another along the lateral direction for anchoring the anchoring device on tissue by said first fixation arrangement and said second fixation arrangement.

The anchoring device comprises a first fixation member and a second fixation member which both shall engage with tissue for anchoring an implantable medical device. Each fixation member comprises a fixation arrangement, for example comprising an arrangement of engaging features such as blades, hooks, spikes, tines, barbs or the like, for engaging with tissue, such that the anchoring is achieved by a co-operation of the fixation members. The fixation members herein are connected to each other by means of a connection structure, the connection structure being configured such that the first fixation member and the second fixation member may be tensioned with respect to one another using the connection structure.

As an alternative to the above aspect, the first fixation member and/or the second fixation member does not comprise a fixation arrangement. Such implantable medical device according to this embodiment, may be advantageously be deployed or used in heart vessels smaller than a ventricle, e.g. an atrium, preferably an atrial appendage.

The fixation members are laterally offset with respect to one another along a lateral direction. The fixation members herein both may be configured to rest on tissue in a planar fashion, wherein a tensioning in between the fixation members is provided by means of the connection structure for holding the fixation members with their fixation arrangements in engagement with tissue.

By providing a tensioning between the first fixation member and the second fixation member using the connection structure the anchoring device may be securely held on tissue, in that the fixation arrangements of the fixation members are held in engagement with tissue.

In addition, an easy repositioning without unnecessary damaging of tissue may become possible, in that a release of the anchoring device from tissue may be achieved by modifying a tensioning in between the fixation members by acting onto the connection structure.

The implantable medical device in particular may be an active implantable medical device and for this may comprise electronic circuitry as well as an energy supply such that the implantable medical device, once implanted into the heart, may perform a control and/or monitoring function within the heart, such as a pacing function and/or a sensing function. The implantable medical device in particular may be a leadless pacemaker device configured for implantation into an atrium of the patient's heart, in particular the right atrium of the patient's heart.

In one embodiment, the implantable medical device comprises an electrode, which may be arranged on a housing of the implantable medical device, or which may be arranged on a lead connected to the implantable medical device, the lead extending from the housing of the implantable medical device to rest on tissue. The implantable medical device may particularly have the shape of a longitudinal capsule having a distal end and a proximal end. The housing herein encapsulates electric and electronic components of the device in a fluid-tight manner, an electrode being placed for example distally on the housing, wherein on the proximal end the housing may for example comprise a coupling device which may be used to establish a coupling to a delivery catheter or the like. The electrode serves to transmit and/or receive electrical signals and for this, when the medical device is implanted into the heart, is in abutment with cardiac tissue.

In one embodiment, the connection structure is configured to tension the first fixation member and the second fixation member towards one another along the lateral direction to cause the first fixation arrangement and the second fixation arrangement to engage with tissue. The connection structure hence provides for a tensioning in between the fixation members by means of which the fixation members are pulled towards each other. In this way the fixation arrangement of the fixation members may be held in engagement with tissue such that the connection structure secures the anchoring of the anchoring device on the tissue. A repositioning may be possible in that the fixation members are displaced with respect to one another along the lateral direction in order to disengage the fixation arrangements from the tissue, such that the fixation members may be removed from the tissue and may be repositioned in order to anchor the implantable medical device on tissue at a different location.

In another embodiment, the connection structure is configured to tension the first fixation member and the second fixation member apart from one another along the lateral direction. Hence, a tensioning in between the fixation members is provided to push the fixation members apart. A release for a repositioning in this embodiment is possible by pushing the fixation members towards one another in order to in this way disengage the fixation arrangements of the fixation members from tissue.

In one embodiment, the connecting structure is elastically deformable and connects, particularly directly connects, the first fixation member and the second fixation member with each other. In one embodiment, the connection structure, the first fixation member and the second fixation member are integrally formed in one piece.

In another embodiment, the connection structure is formed by the housing of the implantable medical device, wherein the first fixation member and/or the second fixation member are movably connected along the lateral direction to each other via the housing particularly via a threaded connection. Particularly, the housing of the implantable medical device may comprise a thread on the surface of the housing, wherein the first fixation member and/or the second fixation member comprise a respective fitting counter thread.

In one embodiment, the first fixation member comprises a first body, the first fixation arrangement protruding from the first body transversely to the lateral direction. Alternatively or in addition, the second fixation member in one embodiment comprises a second body, the second fixation arrangement protruding from the second body transversely to the lateral direction. The fixation arrangements may have the shape of blades, spikes, barbs, tines or other structures which may protrude from the respective body in order to provide for an engagement with tissue. Each fixation member herein may have a generally planar shape and may be placed on tissue such that the fixation arrangement may engage with tissue. For establishing the engagement, the respective fixation member may be gently pressed onto the tissue such that the fixation arrangement comes to engage with the tissue. The anchoring herein is secured by a tensioning provided by means of the connection structure.

In one embodiment, the first fixation arrangement is formed by at least one first engaging element which is arranged at an angle with respect to the first body to generally point towards the second fixation arrangement along the lateral direction. Alternatively or in addition, the second fixation arrangement is formed by at least one second engaging element which is arranged at an angle with respect to the second body to generally point towards the first fixation arrangement along the lateral direction. The at least one first engaging element and/or the at least one second engaging element may be shaped as a blade, a spike, a barb, or a tine for engaging with tissue. The respective engaging element herein is angled with respect to the body of the associated fixation member such that the engaging element generally points towards the respective other fixation member. This is understood to mean that a vector indicating a general direction of extension of the engaging element with respect to the associated body comprises a directional vector component which points towards the other fixation member.

In that the engaging elements of the fixation members generally point towards one another it may be achieved that a tensioning force provided by the connection structure and pulling the fixation members towards each other may hold the engaging elements in engagement with tissue. Particularly, in that the engaging elements are tensioned towards one another, the engaging elements are forced to engage with tissue. By modifying a tensioning provided by the connection structure, in particular by pulling the fixation members apart from one another, the engagement of the engaging elements may be released such that the anchoring device may be removed from the tissue and may for example be repositioned at another location.

Each fixation member may for example be made from a body in the shape of a frame structure carrying the fixation arrangement in the shape of an arrangement of blades, spikes, tines, hooks or the like. The fixation member including the body and the fixation arrangement herein may be formed as a single, integral piece for example from a spring elastic metal material, such as shape memory alloy, for example a nickel titanium alloy, such as a Nitinol material. Alternatively, the fixation member including the body and the fixation arrangement may be made from a biocompatible polymeric, plastic or composite material, e.g. a silicone material.

In one embodiment, one of the first fixation member and the second fixation member comprises a connector element for connecting the anchoring device to a delivery device, the connector element being configured for introducing a tensioning force for tensioning the first fixation member and the second fixation member with respect to one another. If the connector element for example is placed on the first fixation member, the second fixation member may be placed on tissue first and a tensioning in between the fixation members may be caused by pulling on the first fixation member using the delivery device locked to the first fixation member by means of the connector element. The connection of the delivery device to the connector element herein is releasable such that the anchoring device may be released from the delivery device and, in the course of releasing the connection, the fixation members may be tensioned with respect to one another by action of the connection structure, such that a secure engagement of the fixation arrangements of the fixation members with tissue is established.

In another embodiment, wherein the connecting structure is formed or provided by the housing of the implantable medical device of the invention, the connecting structure is designed in form of a first thread and a second thread on the outer surface of the housing, wherein the first thread has an opposite orientation with respect to the second thread, e.g. one left-hand thread and one right-hand thread, and each of the first and second fixation member comprises a respective counter thread configured to form-fittingly engage with the first and second thread on the housing of the implantable medical device, respectively.

However, it is also conceivable in an alternative embodiment that the housing comprises only one thread, and only the first or second fixation member comprises a corresponding form-fitting counter thread, by which the respective fixation member can be rotatable and movable along the lateral direction attached or fixed to housing, while the respective other fixation member is only rotatably attached or fixed to the housing but not movable along the later direction. This may be, for example, achieved by one or more circumferential ribs on the surface of the housing and a corresponding sleeve or nut connected to or comprised within the other fixation member and having a corresponding one or more grooves on the inner surface, in which the one or more ribs can engage. Alternatively, the housing may have one or more circumferential grooves, and the afore-mentioned sleeve or nut have one or more ribs that can engage in the one or more grooves.

In any of the above embodiments, tensioning between the first and second fixation member may be easily achieved by rotating the implantable medical device. For example, the implantable medical device may be placed at the desired location such that the fixation members, particularly their respective fixation engaging elements, engage with the tissue at the desired location. For securing the implantable medical device at the desired location, the implantable medical device is rotated such that the first and second fixation member move towards each other, by which the grip of the engaging elements towards the tissue is increased. For release, the implantable medical device is rotated in the opposite direction, by which the first and second fixation member move apart from one another.

In one embodiment, the implantable medical device is fixedly and non-releasably connected to the anchoring device, such that the implantable medical device is implanted together with the anchoring device and is anchored on tissue using the anchoring device.

In another embodiment, the implantable medical device is to be attached to the anchoring device and may be releasably connected to the anchoring device. In this embodiment, the anchoring device may for example be implanted first without the implantable medical device, wherein the implantable medical device is implanted in a second step and for this is placed on the anchoring device which already is anchored to tissue at a location of interest. The implantable medical device may be released from the anchoring device for example for replacing the implantable medical device.

In an embodiment in which the implantable medical device may releasably be attached to the anchoring device, the anchoring device may comprise a docking mechanism allowing for a (releasable) attachment of the implantable medical device to the anchoring device. The docking mechanism may be designed such that the implantable medical device may be docked onto the anchoring device when the anchoring device already is implanted in a patient, such that an implantation procedure includes the implantation of the anchoring device in a first step and the implantation of the implantable medical device by docking to the anchoring device in a second step.

A docking mechanism of this kind may for example comprise one or multiple magnet elements which are configured to magnetically interact with the implantable medical device to connect the implantable medical device to the anchoring device. A connection of the implantable medical device to the anchoring device hence is established by magnetic attraction forces of an arrangement of magnet elements of the anchoring device and an arrangement of magnet elements of the implantable medical device. The magnet elements herein both on the side of the anchoring device and on the side of the implantable medical device may be permanent magnets, or on one side may be permanent magnets and on the other side may be a magnetically passive armatures, for example in the shape of a ferromagnetic elements which magnetically interact with permanent magnets of the respective other assembly.

In another embodiment, a docking mechanism may comprise hooks and loops or other features for establishing a mechanical, e.g. positive locking connection in between the implantable medical device and the anchoring device, for example a connection of a hookan-loop fastener (Velcro) type.

In another embodiment, the docking mechanism may comprise a threading member into which the implantable medical device may be screwed in order to attach the implantable medical device to the anchoring device. Hence, for placing the implantable medical device on the anchoring device the implantable medical device is screwed into the threading member of the docking mechanism, wherein the connection of the implantable medical device to the anchoring device may be released by unscrewing the implantable medical device from the threading member.

In one embodiment, the connection structure comprises a deformable member connecting the first fixation member and the second fixation member with one another. The deformable member may for example have the shape of an arrangement of struts which connects the first fixation member and the second fixation member to one another, wherein the deformable member may be deformed in order to provide for a tensioning in between the fixation members.

In one embodiment, the deformable member may have a meandering shape such that the deformable member connects the fixation members to one another and is elastically deformable along the lateral direction.

In another embodiment, an arrangement of deformable members in the shape of struts may provide for a connection of the fixation members to a threading member for establishing a screw connection of the anchoring device to the implantable medical device.

A tensioning in between the fixation members may generally be caused by a delivery device, wherein by means of the delivery device the fixation members for example may be displaced with respect to one another such that the connection structure is tensioned and hereinafter provides for a tensioning in between the fixation members in an implanted state of the anchoring device. The tensioning may be established by for example acting onto one of the fixation members, using the delivery device, for displacing the fixation members with respect to one another. Alternatively, the tensioning may be established by acting onto the connection structure in order to deform the connection structure for providing for a tensioning.

In one embodiment, the connection structure comprises a tensioning member configured to act onto the deformable member, the tensioning member being actuatable for deforming the deformable member for tensioning said first fixation member and said second fixation member with respect to one another along the lateral direction. The tensioning member for example may have the shape of a slider which may be actuated in order to deform the connection structure, for example by sliding along deformable members of the connection structure in the shape of struts, wires or wire frames.

The tensioning member, in one embodiment, may be actuated by the delivery device by acting onto the tensioning member. In another embodiment, the tensioning member may be configured to interact with the implantable medical device for tensioning the first fixation member and the second fixation member with respect to one another along the lateral direction. In this embodiment the tensioning by means of the tensioning member is established for example when attaching the implantable medical device to the anchoring device, for example when screwing the implantable medical device into a docking mechanism in the shape of a threading member. Hence, when attaching the implantable medical device to the anchoring device the connection structure is automatically tensioned such that the fixation members are secured in their engagement with tissue.

In one embodiment, at least one of the first fixation member, the second fixation member and the connection structure comprises a radiopaque marker, which facilitates the implantation of the anchoring device. For example, a radiopaque marker may be arranged on each fixation member, such that by means of the radiopaque markers a relative placement of the fixation members with respect to one another can be visualized using a suitable imaging technology, such as an X-ray technique. Particularly, using the radiopaque markers a lateral distance in between the fixation members can be assessed, such that a tensioning in between the fixation members and a proper placement of the fixation members with respect to one another can be evaluated.

Additional radiopaque markers may be provided on the implantable medical device, and/or on the delivery device. For example, by means of radiopaque markers on the delivery device and on a connector element of the anchoring device a relative placement of the delivery device with respect to the anchoring device can be assessed.

According to one aspect, an implantation system comprises an implantable medical device and an anchoring device according to the kind described above. The implantable medical device may be releasably connectable to the anchoring device using a docking mechanism of the anchoring device. It however is also conceivable that the implantable medical device is integrally formed and connected to the anchoring device.

In one embodiment, the implantable medical device extends longitudinally along a longitudinal axis. The implantable medical device may for example have a generally cylindrical shape, a cylinder axis corresponding to the longitudinal axis of the implantable medical device. A housing of the implantable medical device may encapsulate electric and electronic components of the implantable medical device, in particular a processor and an energy storage such as a battery.

The anchoring device herein, in one embodiment, is configured for anchoring the implantable medical device in an orientation in which the longitudinal axis is arranged along the lateral direction, such that the implantable medical device, in an attached state, assumes a generally parallel orientation with respect to the anchoring device. In this embodiment an electrode may for example be arranged on a lead connected to the implantable medical device, the lead with the electrode arranged thereon being configured to rest on tissue.

In another embodiment, the anchoring device is configured for anchoring the implantable medical device in an orientation in which the longitudinal axis is arranged transversely to the lateral direction. In this embodiment, an electrode may be arranged on the housing of the implantable medical device and may come into contact with tissue upon attaching the implantable medical device to the anchoring device.

In another aspect, a method for implanting an implantable medical device using an anchoring device is provided, the method comprising: placing a first fixation member of the anchoring device on tissue, the first fixation member having a first fixation arrangement for engaging with tissue; placing a second fixation member of the anchoring device on tissue, the second fixation member having a second fixation arrangement for engaging with tissue, the second fixation member being laterally offset with respect to the first fixation member along a lateral direction; and tensioning, using an elastically deformable connection structure, said first fixation member and said second fixation member with respect to one another along the lateral direction for anchoring the anchoring device on tissue by said first fixation arrangement and said second fixation arrangement, the connection structure operatively connecting said first fixation member and said second fixation member with each other.

The implantation of the implantable medical device may include, in one embodiment, to first place one of the fixation members on tissue, to then tension the fixation members with respect to one another using the connection structure and to then place the other fixation member on tissue.

In another embodiment, the implantation of the implantable medical device may include to place both fixation members on tissue simultaneously, and to then tension the fixation members with respect to another using the connection structure.

The advantages and advantageous embodiments described above for the implantable medical device equally apply also to the method, such that it shall be referred to the above in this respect.

The various features and advantages of the present invention may be more readily understood with reference to the following detailed description and the embodiments shown in the drawings. Herein,
- Fig. 1: shows a schematic view of the human heart;
- Fig. 2: shows a schematic drawing of an anchoring device for anchoring an implantable medical device on tissue;
- Fig. 3: shows a view of an embodiment of an anchoring device;
- Fig. 4: shows another view of the anchoring device;
- Fig. 5: shows a schematic view of a docking mechanism of the anchoring device;
- Fig. 6: shows a partial view of a delivery device for delivering the anchoring device;
- Fig. 7: shows a front view of the arrangement of Fig. 6;
- Fig. 8A: shows a first step for implanting the anchoring device;
- Fig. 8B: shows another step in the process of implanting the anchoring device;
- Fig. 8C: shows yet another step in the process of implanting the anchoring device;
- Fig. 8D: shows yet another step in the process of implanting the anchoring device;
- Fig. 9: shows another embodiment of an anchoring device;
- Fig. 10: shows a view of a coupling element of an implantable medical device;
- Fig. 11: shows a view of an embodiment of a tensioning element of the anchoring device;
- Fig. 12: shows a view of another embodiment of a tensioning element;
- Fig. 13: shows a view of the anchoring device, while screwing an implantable medical device into the anchoring device;
- Fig. 14: shows the anchoring device in an attached state of the implantable medical device;
- Fig. 15: shows another embodiment of an anchoring device; and
- Fig. 16: shows another embodiment of an anchoring device.

Subsequently, embodiments shall be described in detail with reference to the drawings. In the drawings, like reference numerals designate like structural elements.

It is to be noted that the embodiments are not limiting for the instant disclosure, but merely represent illustrative examples.

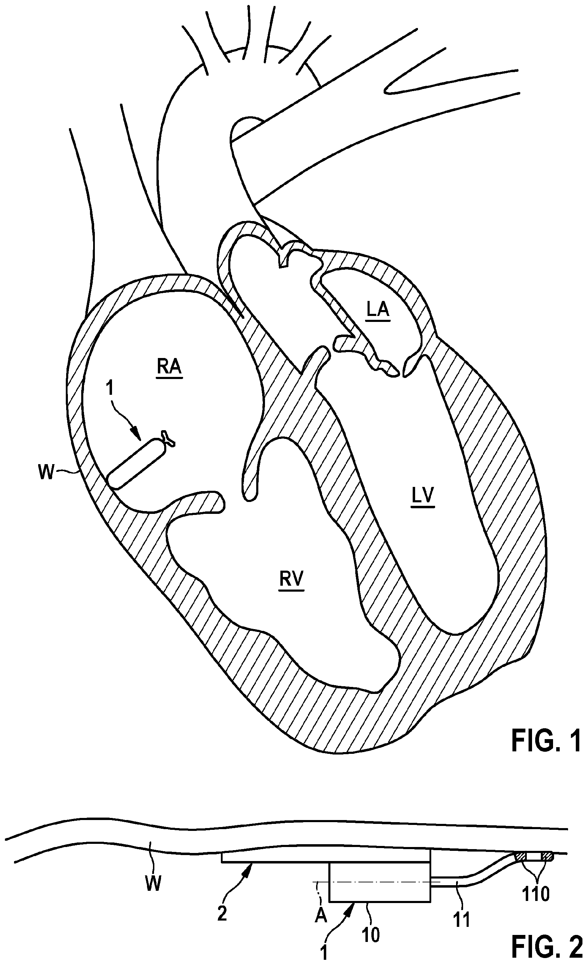

Fig. 1 shows, in a schematic drawing, a human heart comprising a right atrium RA, a right ventricle RV, a left atrium LA and a left ventricle LV, an implantable medical device 1 being implanted for example into the right atrium RA, the implantable medical device 1 e.g. having the shape of a leadless pacemaker device for providing a pacing of the heart's activity at the right atrium RA.

For implantation the implantable medical device 1 by means of a delivery system is placed in a chamber of the heart, for example in a ventricle LV, RV or an atrium LA, RA, and is delivered from the delivery system such that it comes to rest on intra-cardiac tissue, i.e. on an intra-cardiac wall W, and is fastened to intra-cardiac tissue.

Fig. 2 shows, in a schematic drawing, an example of an implantable medical device 1 in the shape of a leadless pacemaker device, the implantable medical device 1 having a housing 10 being configured to be placed on intracardiac tissue, for example an atrial wall W. The implantable medical device 1 has the shape of a capsule extending longitudinally along a longitudinal axis A, the housing 10 encapsulating electrical and electronic components of the implantable medical device 1 therein in a fluid-tight manner.

In the embodiment of Fig. 2, a lead 11 is connected to the housing 10 of the implantable medical device 1, the lead 11 extending from the housing 10 and carrying one or multiple electrodes 110 to be placed on intra-cardiac tissue in order to come into electric contact with an electrical conduction system of the heart. By means of the electrodes 110 electrical signals are to be injected into tissue for providing for a pacing action, or electrical signals shall be received in order to monitor a cardiac activity.

The implantable medical device 1 shall be placed on tissue and shall be anchored on the tissue during its operative lifetime, for example multiple months or even years. For this, an anchoring device 2 is provided which serves to anchor the implantable medical device 1 on tissue in order to in this way hold the implantable medical device 1 in place on the tissue during operation of the implantable medical device 1.

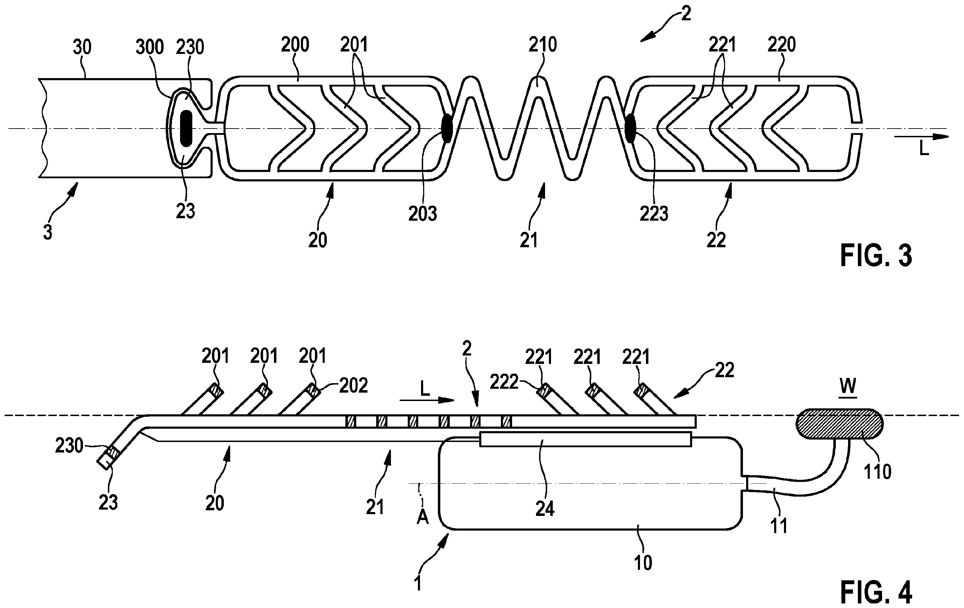

Referring now to Figs. 3 and 4, in one embodiment the anchoring device 2 comprises fixation members 20, 22 which are connected to each other by means of a connection structure 21 having a deformable member 210 extending in a meandering shape in between the fixation members 20, 22. The fixation members 20, 22 serve to connect to tissue such that by means of the fixation members 20, 22 an anchoring on tissue may be established.

The fixation members 20, 22 each comprise a body 200, 220 being formed by a frame structure carrying a fixation arrangement 201, 221 formed by engaging elements 202, 222 in the shape of blades. The engaging elements 202, 222 are arranged, as visible in the side view of Fig. 4, at an angle with respect to the body 200, 220, the engaging elements 202, 222 generally pointing towards each other and being sharpened at their ends such that they may be brought into engagement with tissue in order to provide for an anchoring of the fixation members 20, 22 on tissue.

In the embodiment of Figs. 3 and 4 the engaging elements 202, 222 form blades similar in shape to fish scales. In that the engaging elements 202, 222 generally point towards each other along the lateral direction L (that is, in the side view of Fig. 4 the engaging elements 202, 222 with a directional vector component of a vector of extension point towards each other), the fixation members 20, 22 with their fixation arrangements 201, 221 can be brought into engagement with tissue and can be securely held on tissue by means of the connection structure 21. In particular, for placing the anchoring device 2 on tissue the fixation members 20, 22 may be displaced with respect to one another along the lateral direction L such that the connection structure 21 is elastically tensioned. Upon deployment of the anchoring device 2, then, the connection structure 21 provides for an elastic tensioning such that the fixation members 20, 22 are pulled towards each other along the lateral direction L and the engaging elements 202, 222 hence are biased with respect to one another to engage with tissue.

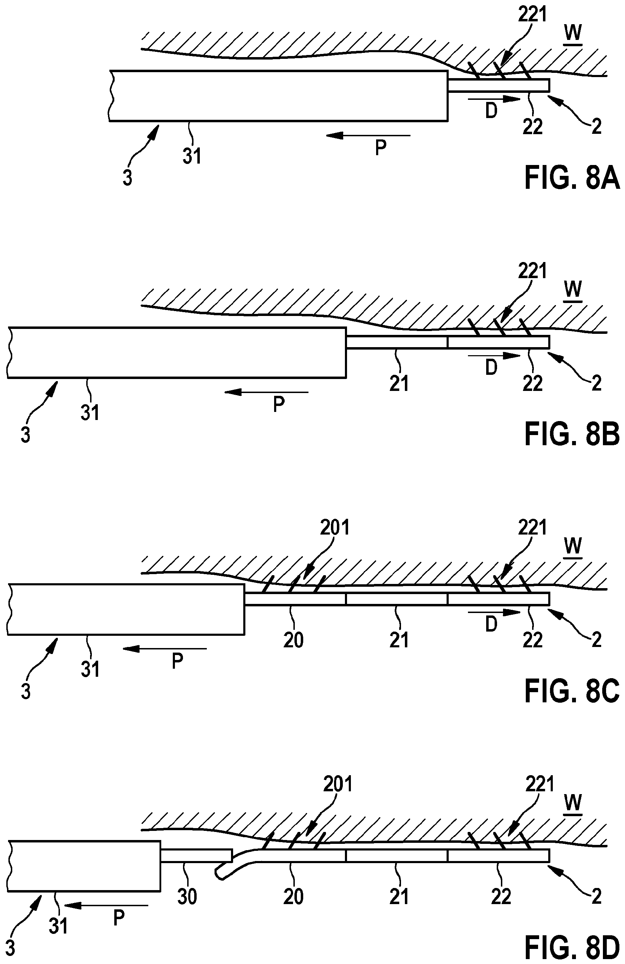

In the embodiment of Figs. 3 and 4 a connector element 23 having an eyelet 230 is formed on the fixation member 20, the connector element 23 serving to establish a connection to a coupling mechanism 30 of a delivery device 3. In particular, by means of the coupling mechanism 30 a connection of the fixation member 20 of the anchoring device 2 to the delivery device 3 may be established in that the connector element 23 engages with a locking feature 300 in the shape of a locking opening, such that a tensioning force may be introduced into the anchoring device 2 upon deployment of the anchoring device 2, as shall subsequently be explained with reference to Figs. 8A to 8D.

In the embodiment of Fig. 4 the implantable medical device 1 is to be releasably attached to the anchoring device 2. For this, the anchoring device 2 comprises a docking mechanism 24 arranged on the fixation member 22, the docking mechanism 24 serving to establish a connection in between the implantable medical device 1 and the anchoring device 2.

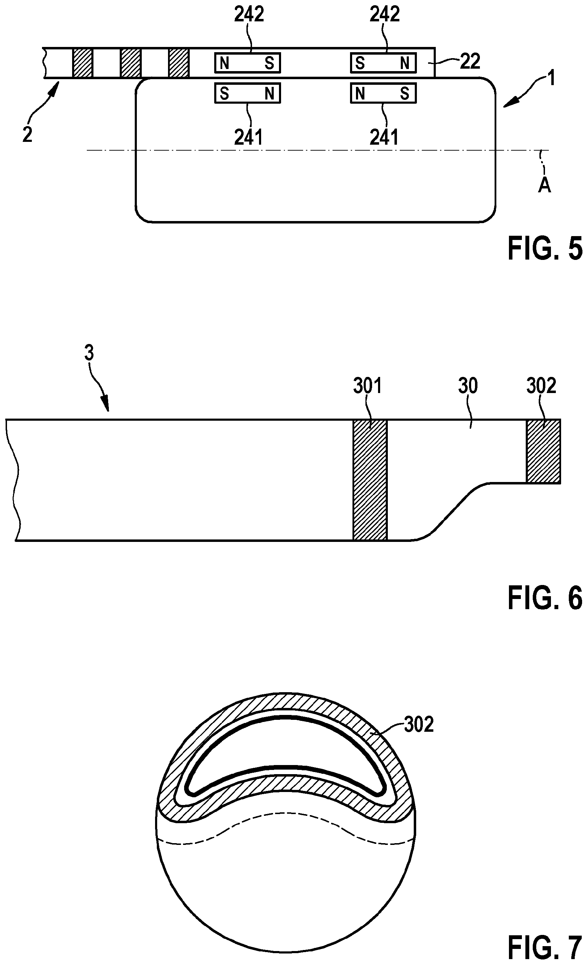

Referring now to Fig. 5, in one embodiment the docking mechanism 24 of the anchoring device 2 comprises magnetic elements 242 in the shape of permanent magnets, which are configured to interact with an arrangement of magnetic elements 241 of the implantable medical device 1 such that the magnet elements 241, 242 magnetically attract each other and hence fix the implantable medical device 1 on the anchoring device 2.

In another embodiment, the docking mechanism 24 may comprise an arrangement of hooks and/or loops to establish e.g. a positive locking connection. For example, the docking mechanism 24 may be configured to establish a Velcro type connection. Features of the docking mechanism 24, for example hooks or loops, may be made of a metal wire material, for example a Nitinol material, or of a plastics material, such as a polymer material.

In order to visualize a process of implanting the anchoring device 2, radiopaque markers 203, 223 may be formed on the anchoring device 2, for example the fixation members 20, 22, as this is shown in Fig. 3. By means of the radiopaque markers 203, 223 for example a lateral distance along the lateral direction L in between the fixation members 20, 22 can be monitored in order to assess a proper positioning of the anchoring device 2 on tissue.

In addition, as shown in Figs. 6 and 7, radiopaque markers 301, 302 may be placed on the delivery device 3, e.g. a catheter. The radiopaque marker 301 herein may be formed for example by a ring extending circumferentially about the generally cylindrical catheter 31. Another marker 302 may be formed at the catheter 3, the marker 302 for example having the shape of a curved ribbon or a mouth such that a rotational orientation of the delivery device 3 may be assessed using a suitable imaging technique, such as an X-ray technique. Referring now to Figs. 8A to 8D, the anchoring device 2 may first be implanted into a patient without an implantable medical device 1 arranged thereon. For the implantation, the anchoring device 2 may be placed on tissue using a delivery device 3 in the shape of a catheter arrangement, a coupling mechanism 30 in the shape of an inner catheter herein being received within an outer sheathing catheter 31.

In a first step, the fixation member 22 may be placed on tissue, as this is shown in Fig. 8A, such that the fixation arrangement 221 of the fixation member 22 engages with tissue. For this, the fixation member 22 is moved out of the catheter 31 in a distal direction D, the engaging elements 222 of the fixation arrangement 221 erecting from the body 220 upon deployment from the catheter 31, such that the engaging elements 222 of the fixation arrangement 221 may engage with tissue.

Having placed the fixation member 22 on tissue, the catheter 31, or a sheath, e.g. an outer sheath, of the catheter 31, respectively, is retracted in a proximal direction P, as shown in Figs. 8B and 8C, such that the connection structure 21 and the fixation member 20 are deployed from the catheter 31. During the deployment herein a pulling force in the proximal direction P is exerted on the connection structure 21, such that the connection structure 21 is tensioned in the proximal direction P, with the fixation member 22 resting on tissue. When the fixation member 20 exits from the catheter 31, the engaging elements 202 of the fixation arrangement 201 erect with respect to the body 200 of the fixation member 20, and the fixation member 20 is gently pressed onto tissue such that the fixation arrangement 201 engages with tissue.

By now further withdrawing the catheter 31, or the sheath, e.g. the outer sheath, respectively, in the proximal direction P the connector element 23 is unsheathed such that it may exit from the locking feature 300 in the shape of the locking opening on the coupling mechanism 30, the anchoring device 2 hence being released from the delivery device 3.

As during the deployment the connection structure 21 is tensioned, after releasing the anchoring device 2 from the delivery device 3 a tensioning in between the fixation members 20, 22 is provided by means of the connection structure 21, the fixation members 20, 22 hence being biased towards each other, such that the engaging elements 202, 222 safely engage with tissue and are reliably held on tissue.

The anchoring device 2 may be made as a single, integral piece from an elastic metal material, such as a shape memory alloy, for example a nickel titanium alloy, for example Nitinol. Alternatively, the anchoring device 2 may be made from a biocompatible polymeric or plastic material such as e.g. a silicone material.

After implanting the anchoring device 2, an implantable medical device 1 may be placed on the anchoring device 2 by docking the implantable medical device 1 to the docking mechanism 24. The implantable medical device 1 herein may easily be replaced by another device, the anchoring device 2 hence providing for a docking station which allows placing and operating different medical devices 1 at a specified location within a patient's heart.

If the anchoring device 2 shall be repositioned, the anchoring to tissue may easily be released by un-hooking the engaging elements 202, 222 from tissue. For this, a delivery device 3 may again be connected to the anchoring device 2 using the coupling mechanism 30, in that the locking feature 300 is brought into engagement and is locked with the connector element 23. By then pulling on the fixation member 20 in the proximal direction P, first the engaging elements 202 of the fixation member 20 may be unhooked, upon which also the fixation member 22 may be released from tissue, with a minimal impact on tissue.

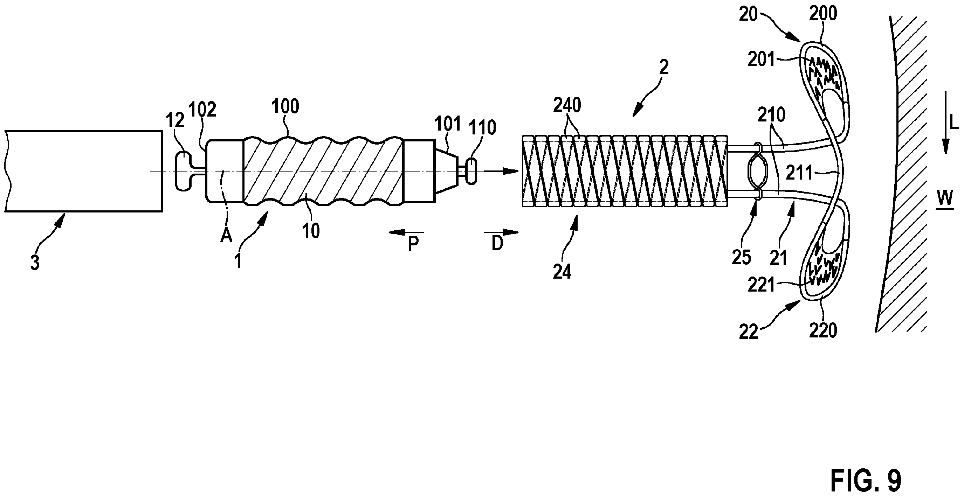

Referring now to Figs. 9 to 14, in another embodiment the anchoring device 2 comprises a docking mechanism 24 having a threading member 240 into which an implantable medical device 1 may be screwed in order to attach the implantable medical device 1 to the anchoring device 2.

In the embodiment of Figs. 2 to 8 the implantable medical device 1 can be attached to the anchoring device 2 such that a longitudinal axis A of the implantable medical device 1 is aligned with the lateral direction L along which the fixation members 20, 22 are offset with respect to one another.

In contrast, in the embodiment of Figs. 9 to 14 the implantable medical device 1 is to be attached to the anchoring device 2 such that the longitudinal axis A of the generally cylindrical implantable medical device 1 is arranged angulated, preferably perpendicularly, to a lateral direction L along which fixation members 20, 22 of the anchoring device 2 are offset with respect to one another.

In the embodiment of Figs. 9 to 14, each fixation member 20, 22 is formed by a body 200, 220 formed by a frame structure carrying a fixation arrangement 201, 221 having a multiplicity of engaging elements 202, 222 such as blades, spikes, tines, hooks or barbs or other engaging features to engage with tissue at a location of interest. A connection structure 21 having deformable connection members 210, 211 in the shape of struts, wires or wire frames serves to operatively connect the fixation members 20, 22 to each other, members 210 herein extending from the fixation members 20, 22 towards the threading member 240 of the docking mechanism 24 and a connection member 211 extending in between the fixation members 20, 22, as this is visible from Fig. 9.

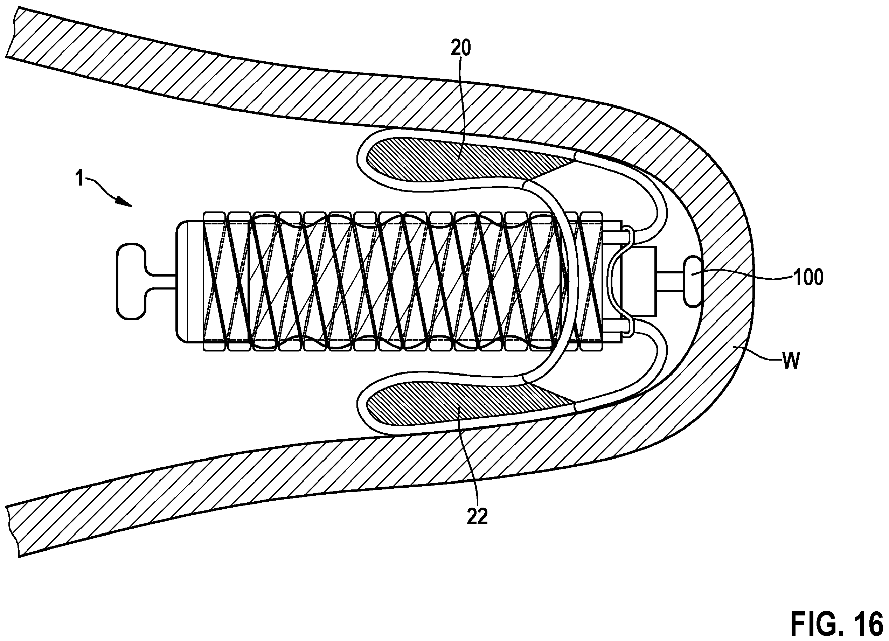

Alternatively, one or both fixation members 20, 22 may be absent of distinct engaging elements 202, 222 as described in the above embodiments. Such embodiment of fixation members 20, 22 may be advantageously used at implantation locations such as the left or right atrial appendage, in which the fixation members 20, 22 can provide a secure fixation by friction or contact pressure only. Such situation is illustrated in Figure 16 in more detail. As it can been seen, for example, in Figure 9, both fixation members 20, 22 extends along an essentially straight line in the unconstrained state. Placing the implantable medical device of the invention in the left or right atrial appendage as shown in Figure 16, forces the fixation members 20, 22 in a constrained bended states, which results in a counter force applied by the fixation members 20 ,22 and acting on the tissue W of the atrial appendage, by which the anchoring device and the implantable medical device is clamped in the atrial appendage. Since the retention force provided by such anchoring device is sufficient in the scenario, additional engaging elements 202, 202 are not necessary, resulting in an even less traumatic anchoring of the implantable medical device of the invention.

The threading member 240 may be formed from one, two or more coils providing for a single-helix, double-helix or multi-helix threading. The implantable medical device 1 on its housing 10 comprises a threading 100 matching the threading of the threading member 240, such that the implantable medical device 1 may be screwed into the threading member 240 in order to attach the implantable medical device 1 to the docking mechanism 24.

A tensioning member 25 is arranged on the deformable members 210 of the connection structure 21, the tensioning member 25 being slidable on the members 210 to approach the fixation members 20, 22 in a distal direction D.

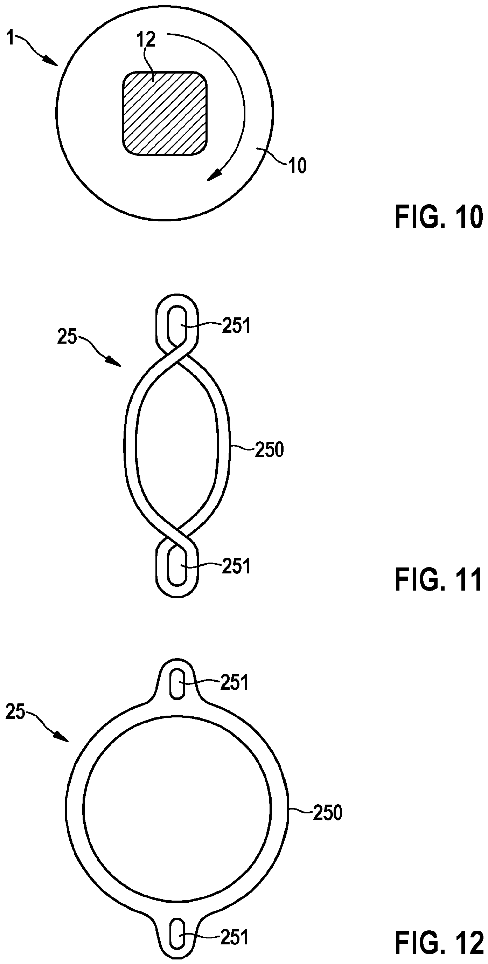

Referring now to Fig. 11, the tensioning member 25 may for example be made from a metal wire, for example from a Nitinol material, the tensioning member 25 forming a body 250 and diametrically opposite eyelets 251. The eyelets 251 are arranged on the deformable members 210 such that the tensioning member 25 may slide on the members 210.

Whereas in the embodiment of Fig. 11 the tensioning member 25 is formed from a wire element, in the embodiment of Fig. 12 the tensioning element 25 is formed from a ring element having a body 250 into which the eyelets 251 are cut. Other than that, the tensioning elements 25 of Figs. 11 and 12 are functionally identical.

The implantable medical device 1 of the embodiment of Fig. 9 comprises a coupling device 12 at a proximal end 102 of the housing 10, the coupling device 12 being formed by a protruding element having a rotationally non-symmetrical shape, as this is shown in Fig. 10. The coupling device 12 may engage with the delivery device 3 such that the implantable medical device 1 may be rotated using the delivery device 3 to screw the implantable medical device 1 in a screwing direction into the threading member 240 of the docking mechanism 24 to attach the implantable medical device 1 to the anchoring device 2.

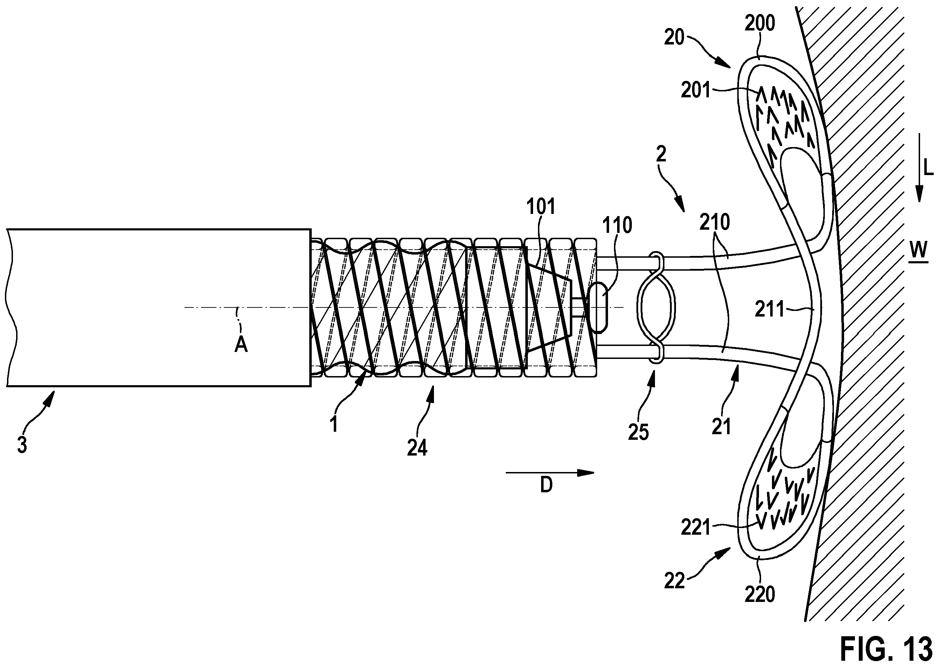

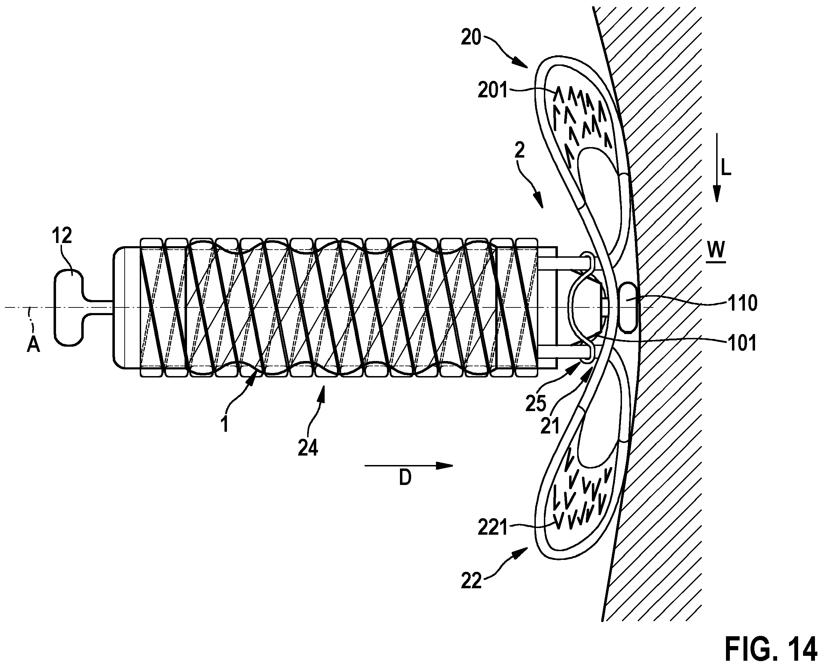

As it is illustrated in Figs. 13 and 14, when screwing the implantable medical device 1 into the docking mechanism 24, the implantable medical device 1 with a distal end 101 is approached towards the tensioning element 25 arranged on the members 210 of the connection structure 21, until the housing 10 of the implantable medical device 1 is brought into engagement with the tensioning member 25 and slides the tensioning member 25 on the members 210 in the distal direction D towards the fixation members 20, 22. This causes the members 210 to be deformed such that the fixation members 20, 22 are pulled towards one another along the lateral direction L, as this is shown in the transition from Fig. 13 to Fig. 14, causing the fixation arrangements 201, 221 of the fixation members 20, 22 to tightly engage with tissue.

In the embodiment of Figs. 9 to 14 the implantable medical device 1 at its distal end 101 comprises an electrode 110, which comes into abutment with tissue at an intra-cardiac wall W when attaching the implantable medical device 1 on the anchoring device 2, as this is shown in Fig. 14. The electrode 110 hence comes into operative connection to an electrical conduction system of the heart, such that the implantable medical device 1 is operative for emitting and/or receiving electrical signals at its implantation side.

For replacing the implantable medical device 1, the implantable medical device 1 may be unscrewed from the anchoring device 2, while leaving the anchoring device 2 in place. The implantable medical device 1 hence may easily be replaced by another medical device using the same anchoring device 2.

For repositioning the anchoring device 2, the tensioning member 25 may be actuated in order to pull the tensioning element 25 in the proximal direction P to release a tensioning on the fixation members 20, 22. Hence, the fixation members 20, 22 are unhooked from tissue and hence may be removed from tissue for repositioning the anchoring device 2.

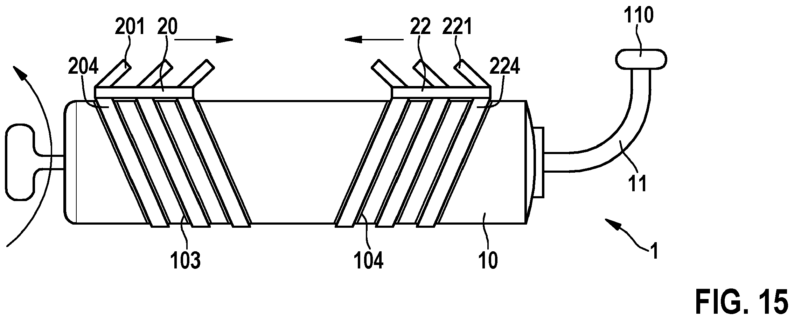

Figure 15 illustrates alternative embodiment of the anchoring device or the implantable medical device 1 of the invention, respectively. In this embodiment, the connecting structure is formed by or comprised within the housing 10 of the implantable medical device 1 of the invention. For this purpose, the surface of the housing comprises two circumferential threads 103, 104, and each of the two fixation members 20, 22 comprises a corresponding fitting counter thread 204, 224 configured to engage in a form-fitting manner with one of the two threads 103, 104 on the surface of the housing. Preferably, each of the threads 204, 224 of the fixation members 20, 22 is formed by or comprises within a sleeve or nut, which is attached or fixed to the respective fixation member 20, 22. The skilled person will appreciate that two threads 103, 104 on the housing as well as the threads 204, 224 of the fixation members 20, 22 have an opposite orientation, respectively, e.g. a left-hand thread/counter thread and a right-hand thread/counter thread. Tensioning of the fixation elements 20, 22 and thereby a secure engagement of the engagement elements 201, 221 with the tissue W can easily be achieved by rotation the implantable medical device 1 of the invention. Likewise, release and disengagement may be achieved by rotating the implantable medical device 1 in the opposite direction.

In addition, there is a pluralities of alternative designs to the above described embodiment conceivable:
- The engaging elements 201, 221 (e.g. fish scales) may be arranged in one longitudinal line or limited area, e.g. along the longitudinal direction L, or all around the circumferential of the thread 204, 225, sleeve or nut, respectively, so that always an engagement element 201, 221 is pointing towards the tissue W. For example, the above-mentioned nut or sleeve may form a fixation member having a plurality of engaging elements 201, 221 circumferentially arranged on the outer surface of the nut or sleeve.
- Each of the fixation members 20, 22 may be independently rotatable form one another, or the fixation members 20, 22 threads 204, 224 may be connected to each other such that they can slide relatively to each other longitudinally but cannot be turned out of relative position ("connection" e.g. built by longitudinal pins in one sleeve or nut and bushings in the other)
- Only one of the fixation members 20, 22 is rotatably and movably along the lateral direction L attached or fixed to the housing with a threaded connection as described above, while the other fixation member is rotatably but not movable along the longitudinal direction L attached or fixed to the housing 10, e.g. via a "thread without pitch", i.e. one or more circumferential ribs on the housing 10 and corresponding one or more fitting grooves on the fixation member, sleeve or nut thereof, respectively, or vice versa.
- orientation of the distal electrode straight or bended (as shown). The latter would need a solution for turning the electrode into the desired position.
- the engaging elements 201, 221 and the structural base they are mounted on may have a large variety of designs, e.g. blades, spikes, tines, hooks or barbs or other engaging features to engage with tissue at a location of interest, and may be directly arranged or fixed on the fixation members 20, 22 or the outer surface of the sleeve or nut or on paddles or on erecting wire loops, etc. However, the fixation members 20, 22 and the engaging elements 201, 221 are preferably designed such that secure engagement and fixation is provided while avoiding an inappropriate trauma for the tissue;

The fixation members 20, 22 advantageously comprise an x-ray-markers, in order to fluoroscopically track the positions. Alternatively, an X-ray marker may be arranged or position near one or both of the fixation members 20, 22.

The anchoring device may extend linearly along the lateral direction or may have a pre-bent shape. The anchoring device may as well stretch out funnel-like on the proximal side to enable a guided alignment of implant and anchoring device (in case both are implanted separately in different clinical procedures). However, a funnel would not be needed when all parts are integrated in the same delivery catheter and being implanted with the implant already partly been screwed into the anchoring device.

Fixation members herein may be shaped to abut tissue in a planar fashion, or may otherwise be shaped to come into contact and engagement with tissue.

Fixation members may, as described above, be securely fastened to tissue by pulling the fixation members towards each other using a suitable connection structure and a tensioning provided by the connection structure. In other embodiments, a fixation may be provided by pushing the fixation members apart from one another, again a tensioning being provided by a connection structure for connecting the fixation members.

An implantable medical device may in either embodiment be arranged on the anchoring device such that a longitudinal axis of the implantable medical device is aligned with a lateral direction along which fixation members are offset with respect to one another. In other embodiments the implantable medical device may be arranged with its longitudinal axis to extend perpendicularly or at an angle to the lateral direction.

The anchoring device may provide for a safe and reliable anchoring on tissue. In embodiments in which the implantable medical device is releasably attached to the anchoring device an easy replacement of medical devices becomes possible.

### LIST OF REFERENCE NUMERALS

- 1: Leadless pacemaker device
- 10: Housing
- 100: Threading
- 101: Distal end
- 102: Proximal end
- 103: Thread element on the housing for securing and tensioning a fixation member
- 104: Thread element on the housing for securing and tensioning a fixation member
- 11: Lead
- 110: Electrode
- 12: Coupling device
- 2: Anchoring device
- 20: Fixation member
- 200: Body (frame)
- 201: Fixation arrangement (engaging element)
- 202: Engaging element
- 203: Marker
- 204: Thread element on the fixation member for securing and tensioning fixation member on the housing21 Connection structure
- 210: Elastic element
- 211: Connection member
- 22: Fixation member
- 220: Body (frame)
- 221: Fixation arrangement (engaging element)
- 222: Engaging element
- 223: Marker
- 224: Thread element on the fixation member for securing and tensioning fixation member on the housing
- 23: Connector element
- 230: Eyelet
- 24: Docking mechanism
- 240: Threading member
- 241, 242: Magnet elements
- 25: Tensioning member
- 250: Body
- 251: Eyelet
- 3: Delivery device
- 30: Coupling mechanism
- 300: Locking feature (locking opening)
- 301,302: Marker
- 31: Catheter
- A: Longitudinal axis
- D: Distal direction
- L: Lateral direction
- LA: Left atrium
- LV: Left ventricle
- P: Proximal direction
- RA: Right atrium
- RV: Right ventricle
- W: Atrial wall

## Claims

1. An anchoring device (2) for anchoring an implantable medical device (1) on tissue, comprising:
a first fixation member (20) optionally having a first fixation arrangement (201) for engaging with tissue;
a second fixation member (22) optionally having a second fixation arrangement (221) for engaging with tissue, the second fixation member (22) being laterally offset with respect to the first fixation member (20) along a lateral direction (L); and
a connection structure (19, 21) operatively connecting said first fixation member (20) and said second fixation member (22) with each other, wherein the connection structure (19, 21) is configured to tension said first fixation member (20) and said second fixation member (22) with respect to one another along the lateral direction (L) for anchoring the anchoring device (2) on tissue by said first fixation arrangement (201) and said second fixation arrangement (221).

2. The anchoring device (2) of claim 1, wherein the connection structure (21) is elastically deformable and connects the first fixation member (20) and the second fixation member (22) with each other, wherein particularly the connection structure (21), the first fixation member (20) and the second fixation member (22) are integrally formed in one piece.

3. The anchoring device (2) of claim 1, wherein the connection structure is formed by or comprised within a housing (19) of the implantable medical device (1), the first fixation member (20) and/or the second fixation member (22) are movably connected along the lateral direction (L) to each other via the housing (10), particularly via a threaded connection (103, 104, 204, 224).

4. The anchoring device (2) of any one of the preceding claims, wherein the connection structure (21) is configured to tension said first fixation member (20) and said second fixation member (22) towards one another along the lateral direction (L) to cause said first fixation arrangement (201) and said second fixation arrangement (221) to engage with tissue.

5. The anchoring device (2) of any one of the preceding claims, wherein said first fixation member (20) comprises a first body (200), said first fixation arrangement (201) protruding from the first body (200) transversely to the lateral direction (L), and/or said second fixation member (22) comprises a second body (220), said second fixation arrangement (221) protruding from the second body (220) transversely to the lateral direction (L).

6. The anchoring device (2) of any one of the preceding claims, wherein said first fixation arrangement (201) is formed by at least one first engaging element (202) which is arranged at an angle with respect to the first body (200) to generally point towards said second fixation arrangement (221) along the lateral direction (L), and/or said second fixation arrangement (221) is formed by at least one second engaging element (222) which is arranged at an angle with respect to the second body (220) to generally point towards said first fixation arrangement (201) along the lateral direction (L).

7. The anchoring device (2) of one of the preceding claims, wherein one of the first fixation member (20) and the second fixation member (22) comprises a connector element (23) for connecting the anchoring device (2) to a delivery device (3), the connector element (23) being configured for introducing a tensioning force for tensioning the first fixation member (20) and the second fixation member (22) with respect to one another.

8. The anchoring device (2) of one of the preceding claims, wherein one of the first fixation member (20) and the second fixation member (22) comprises a docking mechanism (24) for attaching the implantable medical device (1) to the anchoring device (2).

9. The anchoring device (2) of claim 6, wherein said docking mechanism (24) comprises at least one magnet element (242) configured to magnetically interact with the implantable medical device (1) to connect the implantable medical device (1) to the anchoring device (2), or
said docking mechanism (24) comprises a threading member (240) for establishing a screw connection in between the anchoring device (2) and the implantable medical device (1), or
said docking mechanism (24) comprises hooks and loops, or other hook-and-loop fastener like feature for establishing a positive locking connection in between the anchoring device (2) and the implantable medical device (1).

10. The anchoring device (2) of one of the preceding claims, wherein the connection structure (21) comprises a deformable member (210) connecting the first fixation member (20) and the second fixation member (22) with one another.

11. The anchoring device (2) of claim 10, wherein the connection structure (21) comprises a tensioning member (25) configured to act onto the deformable member (210), the tensioning member (25) being actuatable for deforming the deformable member (210) for tensioning said first fixation member (20) and said second fixation member (22) with respect to one another along the lateral direction (L)

12. The anchoring device (2) of claim 11, wherein the tensioning member (25) is configured to interact with the implantable medical device (1) for tensioning said first fixation member (20) and said second fixation member (22) with respect to one another along the lateral direction (L) when attaching the implantable medical device (1) to the anchoring device (2).

13. The anchoring device (2) of one of the preceding claims, wherein at least one of the first fixation member (20), the second fixation member (22) and the connection structure (21) comprises at least one radiopaque marker (203, 223).

14. An implantation system comprising an implantable medical device (1) and an anchoring device (2) according to at least one of the preceding claims.

15. The implantation system of claim 14, that the implantable medical device (1) extends longitudinally along a longitudinal axis (A), wherein the anchoring device (2) is configured for anchoring the implantable medical device (1) in an orientation in which the longitudinal axis (A) is arranged along the lateral direction (L) or transverse to the lateral direction (L).

16. A method for implanting an implantable medical device (1) using an anchoring device (2), the method comprising:
placing a first fixation member (20) of the anchoring device (2) on tissue, the first fixation member (20) having a first fixation arrangement (201) for engaging with tissue;
placing a second fixation member (22) of the anchoring device (2) on tissue, the second fixation member (22) having a second fixation arrangement (221) for engaging with tissue, the second fixation member (22) being laterally offset with respect to the first fixation member (20) along a lateral direction (L); and
tensioning, using an elastically deformable connection structure (21), said first fixation member (20) and said second fixation member (22) with respect to one another along the lateral direction (L) for anchoring the anchoring device (2) on tissue by said first fixation arrangement (201) and said second fixation arrangement (221), the connection structure (21) operatively connecting said first fixation member (20) and said second fixation member (22) with each other.
